# EUROPÄISCHE PATENTANMELDUNG

(11) **EP 1 889 597 A1**
(43) Veröffentlichungstag der Anmeldung: **20.02.2008**
(21) Anmeldenummer: 07014360.7
(22) Anmeldetag: 21.07.2007
(51) Int. Cl.: A61K 8/06, A61K 8/34, A61K 8/36, A61K 8/55, A61K 8/67, A61K 31/375, A61K 31/045, A61K 31/20, A61K 31/6615, A61Q 1/00, A61Q 19/08, A61Q 19/10

(54) **Wirkstoffkombinationen aus Ascorbylverbindung und hydriertem Lecithin**

(30) Priorität: 28.07.2006 DE 102006035040
(71) Anmelder: Beiersdorf AG, 20253 Hamburg (DE)
(72) Erfinder: Kruse, Uta, 20149 Hamburg (DE); Riedel, Heidi, 22083 Hamburg (DE); Warnke, Katja Dr., 22547 Hamburg (DE); Fischer, Britta, 22880 Wedel (DE)

(57) **Zusammenfassung**

Wirkstoffkombinationen aus
a) einem oder mehreren Wirkstoffen, gewählt aus der Gruppe der Ascorbinsäure und der Ascorbylverbindungen
und
b) Abmischungen aus C₁₂-C₁₆ Alcohol, Hydriertem Lecithin und Palmitinsäure.

## Beschreibung

Die vorliegende Erfindung betrifft kosmetische Zubereitungen auf der Basis von Öl-in-Wasser-Formulierungen, welche sich durch einen erhöhten Gehalt an Polyolen auszeichnen. Insbesondere betrifft die vorliegende Erfindung kosmetische Zubereitungen zum Schminken der Haut.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Die vorliegende Erfindung betrifft ferner antioxidativ wirksame Wirkstoffkombinationen, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend.

Antioxidantien sind chemische Substanzen unterschiedlichster Struktur, die unerwünschte, durch Sauerstoff-Einwirkungen und andere oxidative Prozesse bedingte Veränderungen in den zu schützenden Stoffen hemmen oder verhindern. Antioxidantien werden insbesondere eingesetzt, um organische Produkte zu schützen, namentlich Fette und Öle, aber auch Wirk- und Zusatzstoffe wie beispielsweise Aromastoffe und dergleichen. Als Antioxidantien wirksam sind beispielsweise durch sterisch hindernde Gruppen substituierte Phenole, Hydrochinone, Brenzcatechine und aromatische Amine sowie deren Metall-Komplexe. Für Fette, Lebensmittel und insbesondere auch kosmetische und dermatologische Zubereitungen eignen sich Tocopherol, Butylhydroxyanisol (BHA), Butylhydroxytoluol (BHT), Octyl- und Dodecylgallat sowie Ascorbin-, Milch-, Citronen- und Weinsäure sowie deren Salzeln einer bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung.

An sich vorzügliches Antioxidantien werden gewählt aus der Gruppe der Ascorbinsäure und der Ascorbylverbindungen.

L-Ascorbinsäure {(R)-5-[(S)-1,2-Dihydroxyethyl]-3,4-dihydroxy-5-H-furan-2-on, Vitamin C} zeichnet sich durch die Strukturformel 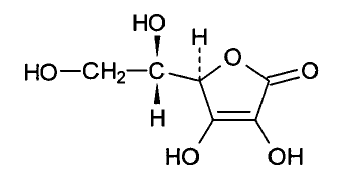 aus. Sie ist leicht löslich in Wasser, gut löslich in Alkohol, unlöslich in Ether, Petrolether, Chloroform, Benzol sowie in Fetten u. fetten Ölen. Ascorbinsäure ist ein Endiol und wirkt als Redukton stark reduzierend. Ascorbinsäure ist wärmeempfindlich und wird insbesondere in Gegenwart von Schwermetallspuren sowie in alkalischem Milieu durch Licht und Luftsauerstoff zersetzt, in reinem, trockenem Zustand ist sie dagegen relativ beständig gegen Licht, Luft und Wärme.

In kosmetischen und dermatologischen Zubereitungen werden anstatt der Ascorbinsäure oftmals Ascorbylverbindungen, bevorzugt Ascorbylester von Fettsäuren, besonders bevorzugt Ascorbylpalmitat eingesetzt, da die Empfindlichkeit dieser Verbindungen auf oxidativen Einfluß gegenüber der Ascorbinsäure stark herabgesetzt ist und diese Verbindungen zumeist besser öllöslich sind, was galenische Vorteile bieten kann.

Ascorbylverbindungen im engeren Sinne sind insbesondere Ascorbinsäuresalze, aber auch die Ascorbylester der allgemeinen Struktur 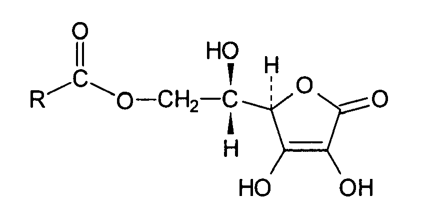 wobei R einen verzweigten oder unverzweigten Alkylrest mit bis zu 25 Kohlenstoffatomen darstellen kann. R kann auch einen Glycosidrest, insbesondere einen Glucosidrest bedeuten.

Eine Aufgabe der vorliegenden Erfindung war, die Nachteile des Standes der Technik zu beseitigen. Insbesondere sollten Wirkstoffe bzw. Zubereitungen, solche Wirkstoffe enthaltend, zur Verfügung gestellt werden, bei deren Verwendung die Schädigung der Haut und/oder des Haares durch oxidativen Einfluß zumindest gemindert, wenn nicht gänzlich verhindert werden können.

In einer besonderen Aufgabenstallung sollten Wege aufgefunden werden, Ascorbylverbindungen, insbesondere Vitamin C und Vitamin-C-Ester gegen schädlichen oxidativen Einfluß zu schützen, und zwar bevorzugt in kosmetischen oder dermatologischen Zubereitungen.

Überraschend hat sich gezeigt, daß Wirkstoffkombinationen aus
a) einem oder mehreren Wirkstoffen, gewählt aus der Gruppe der Ascorbinsäure und der Ascorbylverbindungen
   und
b) Abmischungen aus C₁₂-C₁₆ Alcohol, Hydriertem Lecithin und Palmitinsäure diese Aufgaben zu lösen vermögen.

Insbesondere sind kosmetische Zubereitungen vom Typ Öl-in-Wasser, welche Wirkstoffkombinationen aus
a) einem oder mehreren Wirkstoffen, gewählt aus der Gruppe der Ascorbinsäure und der Ascorbylverbindungen
   und
b) Abmischungen aus C₁₂-C₁₆ Alcohol, Hydriertem Lecithin und Palmitinsäure enthält, vorteilhafte Ausführungsformen der vorliegenden Erfindung.

Insbesondere vorteilhaft im Sinne der vorliegenden Erfindung ist auch die Verwendung von Abmischungen aus C₁₂-C₁₆ Alcohol, Hydriertem Lecithin und Palmitinsäure zur Stabilisierung von Wirkstoffen, gewählt aus der Gruppe der Ascorbinsäure und der Ascorbylverbindungen gegen Oxidation.

Es war für den Fachmann daher nicht vorauszusehen gewesen, daß die erfindungsgemäß verwendeten Wirkstoffkombinationen bzw. kosmetische Zubereitungen, solche enthaltend
- besser als Antioxidans wirken
- besser als Radikalfänger wirken
- besser die Bindung von schädlichen Photoprodukten an Lipide, DNS und Proteine verhindern
- besser gegen die Hautalterung wirken
- besser die Haut gegen Photoreaktionen schützen
- besser entzündlichen Reaktionen vorbeugen würde
als die Wirkstoffe, Wirkstoffkombinationen und Zubereitungen des Standes der Technik. Ferner war nicht vorauszusehen gewesen, daß die erfindungsgemäß verwendeten Wirkstoffkombinationen in kosmetischen oder dermatologischen Zubereitungen höhere Stabilität aufweist als die jeweils einzeln verwendeten Wirkstoffe, was insbesondere die Ascorbylverbindungen und ganz besonders das Vitamin C betrifft.

Zur Anwendung werden die erfindungsgemäßen Zubereitungen in der für Kosmetika üblichen Weise auf die Haut in ausreichender Menge aufgebracht. Sie können wie üblich zusammengesetzt sein und zur Pflege der Haut und als Schminkprodukt in der dekorativen Kosmetik dienen.

Vorteilhafte Ausführungsformen der vorliegenden Erfindung werden beim Einsatz von Ascorbinsäure selbst, aber auch von Ascorbylverbindungen, gewählt aus der Gruppe Ascorbylpalmitat, Natriumascorbylphosphat, Magnesiumascorbylphosphat, Zinkascorbylphosphat, Ascorbyl-2-glucosid erhalten.

Abmischungen aus C₁₂-C₁₆ Alcohol, hydriertem Lecithin und Palmitinsäure werden unter der INCI-Bezeichnung Hydrogenated Lecithin eingesetzt und sind beispielsweise unter der Handelsbezeichnung Biophilic H von der Fa. Lucas Meyer erhältlich.

Die erfindungsgemäßen kosmetischen oder dermatologischen Zubereitungen können wie üblich zusammengesetzt sein und zur Behandlung, der Pflege und der Reinigung der Haut und/oder der Haare und als Schminkprodukt in der dekorativen Kosmetik dienen.

Sie enthalten bevorzugt 0,001 Gew.-% bis 10 Gew.-%, bevorzugt 0,05 Gew.-% bis 5 Gew.-%, insbesondere 0,005 - 1,0 Gew.-%, bezogen auf das Gesamtgewicht der Zubereitungen, an einem oder mehreren Wirkstoffen, gewählt aus der Gruppe der Ascorbinsäure und der Ascorbylverbindungen.

Sie enthalten ferner bevorzugt 0,1 Gew.-% bis 15 Gew.-%, bevorzugt 0,5 Gew.-% bis 7,5 Gew.-%, insbesondere 1,0 - 5,0 Gew.-%, bezogen auf das Gesamtgewicht der Zubereitungen, an Abmischungen aus C₁₂-C₁₆ Alcohol, Hydriertem Lecithin und Palmitinsäure, insbesondere einem Produkt, welches nach INCl "Hydrogenated Lecithin" genannt und unter der Bezeichnung "Biophilic H" von der Fa. Lucas Meyer vertrieben wird.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindugsgemäß vorteilhaft, Folsäure und/oder deren Derivate in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, Folsäure und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Menge an Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1-10 Gew.-%, bezogen auf das Gesamtgewicht der Zubereitung.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; Ether von Fettalkoholen wie vorzugsweise Dicaprylyl Ether, Carbonate von Fettalkoholen wie vorzugsweise Dicaprylyl Carbonate, Wachse wie Hydrogenated Cocoglycerides, Mristyl Myristate, Myristyl Lactate, Caprylic/Capric/Isostearic/Adipic Triglyceride, Shea Butter, Stearyl Dimethicone, Hydrogenated Vegetable Oil. Besonders bevorzugt sind Shea Butter, Myristyl Lactate.
- Alkylbenzoate;
- Siliconöle wie Cyclomethicone , Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Hydroxypropylmethylcellulose, Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Besonders vorteilhaft ist der Einsatz von Xanthangummi.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat und / oder Xanthan Gummi ist.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die erfindungsgemäßen Zubereitungen können vorteilhaft auch anorganische oder organische Pigmente enthalten.

Bevorzugt im Sinne der vorliegenden Erfindung sind Pigmentmischungen aus Weiß-Pigmenten (z. B. Kaolin, Titandioxid oder Zinkoxid) und anorganischen Farbpigmenten (z. B. Eisenoxid-Pigmente, Chromoxide), wobei die Pigmente beschichtet ("gecoatet") oder unbeschichtet vorliegen können. Unter den Farbpigmenten sind Eisenoxide besonders bevorzugt.

Weißpigmente sind Pigmente, deren optische Wirkung vorwiegend auf nicht-selektiver Lichtstreuung beruht (siehe auch DIN 55944: 2003-11). Von den chemisch oft sehr ähnlichen Füllstoffen unterscheiden sich anorganische Weißpigmente vor allem durch ihre im Allgemeinen höhere Brechzahl und - damit verbunden - ihr höheres Streuvermögen sowie nach DIN 55943: 2001-10 durch ihre Anwendung.

Erfindungsgemäß bevorzugte Weißpigmente zeigen keine Absorption im Bereich des sichtbaren Lichts, dafür aber ein hohes Streuvermögen, welches ein hohes Deckvermögen zur Folge hat. Das Streuvermögen ist umso größer, je größer die Differenz zwischen der Brechzahl des Weißpigmentes und der des umgebenden Mediums ist.

Erfindungsgemäß vorteilhafte Weißpigmente sind Titandioxide (Brechzahlen: 2,55 für Anatas und 2,75 für Rutil) und Zinkoxide (Brechzahl zwischen 1,95 und 2,1). Besonders bevorzugt ist Titandioxid.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäß vorteilhaft zusätzlich zu einem oder mehreren Weiß- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenförmiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenförmige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für erfindungsgemäß bevorzugte handelübliche Effektpigmente sind: Timiron® von Merck , Iriodin® von Merck (Perl- und Farbglanzpigmente für dekorative technische Anwendungen), Xirallic® von Merck (farbintensive Kristalleffektpigmente).

Ferner können die erfindungsgemäßen Zubereitungen vorteilhaft auch organische Farbpigmente enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weißpigmente sind ohne praktische Bedeutung.

Die Pigmente können vorteilhaft im Sinne der vorliegenden Erfindung auch in Form kommerziell erhältlicher öliger oder wässriger Vordispersionen zur Anwendung kommen.

Es ist ferner vorteilhaft im Sinne der vorliegenden Erfindung, wenn die erfindungsgemäße Zubereitung einen oder mehrere Farbstoffe enthält.

Die Farbstoffe können sowohl synthetischen als auch natürlichen Ursprungs sein.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) sowie Lauroyl Lysine, Polymethylsilesquioxane, Polymethylmethacrylate, Polymethylmethacrylate Crosspolymer, Nylon, Talkum, beschichtetes Talkum, z.B. mit Dimethicone und Trimethylsiloxysilicate, Mica, Silica

Feine Fältchen sieht man, weil sich die Lichtreflexion auf der Hautoberfläche von der in den Faltensenken unterscheidet. Es ist daher vorteilhaft im Sinne der vorliegenden Erfindung, lichtstreuende Pigmente einzusetzen, die die Faltentiefe optisch reduzieren, indem sie den Anteil an diffus reflektiertem Licht in der Falte reduzieren, wodurch die Haut glatter und natürlicher aussieht. Auf diese Weise lässt sich auch übermäßiger Glanz der Haut optisch reduzieren. Vorteilhaft zu diesem Zweck zu verwenden ist speziell beschichtetes Silica - z. B. ganz besonders vorteilhaft Silica LDP (Silica + Titanium Dioxide + Iron Oxides), welches unter dem Handelsnamen Ronasphere LDP von Merck erhältlich ist.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Verdickungsmittel, anfeuchtende und/oder feuchthaltende Substanzen, Füllstoffe, die das Hautgefühl verbessern, oder andere übliche Bestandteile einer kosmetischen Formulierung wie Alkohole, Polyole, Polymere, Schaumstabilisatoren, Elektrolyte, organische Lösungsmittel oder Siliconderivate.

Geeignete Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc.

Obwohl die genannten Konservierungsmittel an sich in den erfindungsgemäßen Zubereitungen vorteilhaft eingesetzt werden können, sind ganz besonders bevorzugt solche Zubereitungen, welche frei von Parabenen und/oder frei von Formaldehydabspaltern sind.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminòdisuccinat VP OC 370 (ca. 30% ige Lösung) und Baypure CX 100 fest erhältlich ist.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen

### Beispiele:

### O/W-Emulsionen und O/W-Foundation

| | **1** | **2** | **3** | **4** | **5** | **6** |
|---|---|---|---|---|---|---|
| Hydriertes Lecithin, C₁₂-C₁₆ Fettalkohol, Palmitinsäure¹ | 1,00 | 5,00 | 3,00 | 2,00 | 2,50 | 4,00 |
| Ethylhexyl Methoxycinnamat | | 3,50 | | | | |
| Octocrylen | | | | | | 2,50 |
| Titandioxid T 805 | | 1,50 | | | | |
| C₁₂₋₁₅ Alkyl Benzoat | | | | | | 7,00 |
| Dicaprylylether | | | 3,50 | 1,00 | 4,00 | |
| Butylenglycol Dicaprylat/ Dicaprat | 2,00 | | 1,50 | 1,00 | | 2,50 |
| Caprylic/Capric Triglycerid | | 3,50 | 2,00 | 2,00 | 4,00 | 2,00 |
| Cetearyllsononanoate | | 2,00 | | | 2,00 | 2,00 |
| Dimethicon | 5,00 | 4,50 | 4,00 | 8,00 | 5,00 | 4,00 |
| Cyclomethicon | 5,00 | 7,00 | | 8,00 | 7,5 | 2,50 |
| Dimethicon / Vinyl Dimethicon Crosspolymer | 4,00 | | | | | 0,50 |
| Squalane | | 2,00 | | 1,50 | | 4,00 |
| Myristyl Lactat | 1,50 | 2,50 | | | | 2,00 |
| Shea Butter | 1,50 | 0,50 | | 4,00 | | 2,00 |
| Polymethylsilesquioxan | | 2,00 | | | | |
| Lauroyl Lysin | | 3,00 | | | | 1,00 |
| Titandioxid (Cl 77891) | 9,00 | 6,00 | | | | 10,00 |
| Pigmente (Cl77492, 77491,77499,77007) | 2,0 | 3,0 | | | | 3,0 |
| Effektpigmente (z.B. beschichtetes Mica)² | | 1,00 | | | | 0,27 |
| Glycerin | 3,00 | 7,50 | | 5,00 | | 10,00 |
| Butylenglycol | 7,00 | 5,00 | | | 7,00 | |
| Vitamin E Acetat | | 0,5 | 1,00 | 0,25 | 0,4 | 0,5 |
| Vitamin C und Derivate / Verbindungen | 0,50 | 0,1 | 0,25 | 0,15 | 0,1 | 1,00 |
| Xanthan Gummi | 0,5 | 0,30 | 0,3 | 0,1 | 0,5 | 0,1 |
| Magnesium Aluminium Silikate | | 3,00 | | 1,00 | | 0,50 |
| DMDM Hydantoin | | 0,60 | 0,40 | | | |
| Parabene | 0,5 | | 0,25 | 0,70 | | 0,70 |
| Phenoxyethanol | 1,00 | 0,40 | | 0,90 | | 0,60 |
| EDTA oder Tetra Natrium lminodisuccinate | | 0,20 | 0,35 | 0,02 | | 0,03 |
| Alkohol | 5,00 | | 10,00 | 3,00 | | |
| Parfüm | 0,20 | 0,20 | | | 0,30 | 0,40 |
| Wasser | ad 100 | ad 100 | ad 100 | ad 100 | ad 100 | ad 100 |

| | | | | | | |
|---|---|---|---|---|---|---|
| ¹ Biophilic H von der Fa. Lucas Meyer ² z.B.. Timiron von Merck (Mica & Cl 77891) | | | | | | |

## Patentansprüche

1. Wirkstoffkombinationen aus
a) einem oder mehreren Wirkstoffen, gewählt aus der Gruppe der Ascorbinsäure und der Ascorbylverbindungen
und
b) Abmischungen aus C₁₂-C₁₆ Alcohol, hydriertem Lecithin und Palmitinsäure.

2. Wirkstoffkombinationen nach Anspruch 1, **dadurch gekennzeichnet, daß** als Ascorbylverbindungen Ascorbinsäuresalze und/oder Ascorbylester der allgemeinen Struktur 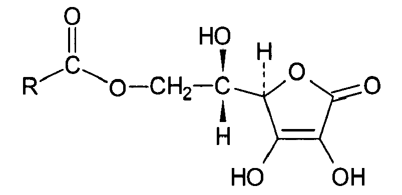 gewählt werden, wobei R einen verzweigten oder unverzweigten Alkylrest mit bis zu 25 Kohlenstoffatomen darstellen kann. R kann auch einen Glycosidrest, insbesondere einen Glucosidrest bedeuten.

3. Wirkstoffkombinationen nach Anspruch 1, **dadurch gekennzeichnet, daß** die Ascorbylverbindungen aus der Gruppe Ascorbylpalmitat, Natriumascorbylphosphat, Magnesiumascorbylphosphat, Zinkascorbylphosphat, Ascorbyl-2-glucosid gewählt werden.

4. Kosmetische Zubereitungen vom Typ Öl-in-Wasser, enthaltend Wirkstoffkombinationen nach einem der vorgehenden Ansprüche.

5. Zubereitungen gemäß Anspruch 4, enthaltend 0,001 Gew.-% bis 10 Gew.-%, bevorzugt 0,05 Gew.-% bis 5 Gew.-%, insbesondere 0,005 - 1,0 Gew.-%, bezogen auf das Gesamtgewicht der Zubereitungen, an einem oder mehreren Wirkstoffen, gewählt aus der Gruppe der Ascorbinsäure und der Ascorbylverbindungen.

6. Zubereitungen gemäß Anspruch 4 oder 5, enthaltend 0,1 Gew.-% bis 15 Gew.-%, bevorzugt 0,5 Gew.-% bis 7,5 Gew.-%, insbesondere 1,0 - 5,0 Gew.-%, bezogen auf das Gesamtgewicht der Zubereitungen, an Abmischungen aus C₁₂-C₁₆ Alcohol, Hydriertem Lecithin und Palmitinsäure.

7. Verwendung von Abmischungen aus C₁₂-C₁₆ Alcohol, Hydriertem Lecithin und Palmitinsäure zur Stabilisierung von Wirkstoffen, gewählt aus der Gruppe der Ascorbinsäure und der Ascorbylverbindungen gegen Oxidation, zum Schutz der Haut gegen Hautalterung, gegen Photoreaktionen, zur Vorbeugung gegen entzündliche Reaktionen
